# EUROPEAN PATENT APPLICATION

(11) **EP 2 339 488 A1**
(43) Date of publication of application: **29.06.2011**
(21) Application number: 09180373.4
(22) Date of filing: 22.12.2009
(51) Int. Cl.: G06F 19/00

(54) **A system for analysis and report of drug interactions**

(71) Applicant: Apteekide Infotehnoloogia OÜ, 50112 Tartu (EE)
(72) Inventor: Uibokand, Siim, 50110, Tartu (EE); Zarkovski, Alexander, 50110, Tartu (EE)
(74) Representative: Plougmann & Vingtoft A/S

(57) **Abstract**

A method for collecting, integrating, analysing and processing medical information from a variety of sources so as to generate drugs interaction analyses and reports to provide doctors, physicians, pharmacists, general practitioners with a tool assisting their decisions on drugs prescription or delivery to a patient.

## Description

### FIELD OF THE INVENTION

The present invention relates to the filed of data analysis and information processing and reporting. In particular to a method for collecting, integrating and analysing and processing data from a variety of sources so as to generate drugs interaction analyses and reports.

### BACKGROUND OF THE INVENTION

Drug interactions occur when the effect of a drug changes due to the co-administration of another drug. Drug-drug interactions, also referred as drugs interaction within the text, DDIs, are a huge problem as they the side-effects may caused by such interaction may lead to the hospitalization of the patient. It is believed that many DDIs may be predicted and avoided, which means they may be targeted both during studies and intervention. Drug interactions may cause considerable side effects.

Epidemiological studies have revealed that the frequency of clinically important interactions is approximately 8.8% (Puckett et al, 1971). Other studies have stated the frequency of drug interactions to be 4.1% (Rupp et al, 1992). A study that was conducted recently among the Swedish population showed that 4% of DDI appeared with elderly people (Johnell and Klarin, 2007).

One other recent study has provided some relatively interesting results regarding the frequency of DDIs with elderly ambulatory patients in six different European countries, also enabling to describe variations between the countries. The data pertaining to the use of the drugs was retrieved from 1,601 elderly people living in six different European countries. The people involved participated during 18 months in a monitored intervention study during which the effect of drugs was evaluated. The results indicated that the elderly used approximately seven drugs per person, 46% took at least one combination with potential interactions. The frequency of potential DDI per person was 0.83 on the average. Almost 10% of the potential DDIs were classified as avoidable under the Swedish interactions classification system but nearly one-third could have been prevented in the case of predisposed patients only. Potential DDIs made the appearance of sub therapeutic effects as feasible as the risk of side effects. The study showed that the potential DDIs are especially frequent with the elderly patients who use many different drugs simultaneously. Some drug combinations have negative effects and therefore more attention should be paid to the discovery and monitoring of the patients using such combinations (Björkman et al, 2002).

Another study was aimed at evaluating age-related variations of clinically important potential DDIs (pDDIs) with ambulatory dyslipidemia patients treated with HMG-CoA reductase inhibitor (statin). The total of 2,742 dyslipidemia patients treated with statin participated in the study. Medications treatment was screened for clinically important pDDI with the help of the electronic drug interactions program (DRUG-REAX System). The study sample comprised 483 (17.6%) patients of 54 years of age or younger, 732 (26.7%) patients between 55-64 years of age, 924 (33.7%) patients between 65-74 years of age and 603 (22.0%) patients of 75 years of age or older. People of 75 years of age or older were prescribed considerably more drugs than those of 54 years of age or younger (the average was 5.8 vs. 3.8 respectively, p < 0,001). Cardio-vascular diseases like the coronary heart disease, cardiac insufficiency or arrhythmia were also more frequent with the patients of 75 years of age and older than those who were younger. The pDDI was also found more frequent with the increase of age: with people of 54 years of age or younger the respective indicator was 7.9% and with the patients of 75 years of age and older 18.4% (p < 0,001). The risk factors of pDDI in case of the patients of 75 years of age and older included: arrhythmia, cardiac insufficiency and co-administration of many drugs. Frequent prescribing of pDDI-inducing cardio-vascular drugs for the patients of 75 years of age and older was in this age group the most common cause for Statin-related or Statin non-related pDDI. Compared to the younger patients, the elder dyslipidemia patients had the highest risk of clinically important pDDI mainly because they are prescribed more drugs. Also, the patients of 75 years of age and older were also prescribed more potentially DDI-risky drugs, especially medications used for treating arrhythmia and cardiac insufficiency. The pDDI-related side effects risk can oftentimes be reduced with the adjustment of dosages, careful monitoring of the patient or some alternative choice of a drug (Egger et al, 2007).

The general problem that appeared from these and other studies is that it is hard to be able to discriminate between potential interactions and clinically proven interactions. The problem is fairly complicated as not all adverse drug reactions (ADRs) and drug interactions (DIs) are easily recognizable for the doctors and also because ADRs are not always reported. The frequency depends on the age of the patient, co-appearing illnesses and many other aspects. Therefore, none of the studies have so far been able to determine the actual frequency of DIs, as well as the connection of DIs to the patient conditions, e.g. age.

There is a large number of publications indicating the significance of the drug interactions problem and reflecting the general interest of the scientists and medical community on the issue.

The above discussed studies identify that there is a need for a convenient, simple and effective method for collecting, analysing, integrating, processing and reporting data on drugs performance and interactions, while at the same time providing a high level of statistical significance and in-depth variable analysis.

Additionally, there is a need for more effective efficient means of collecting, analysing, integrating, processing and reporting data on drugs performance and interactions in relation to related data of the patient, e.g. health or age related.

Further, there is a need for more effective efficient means of collecting, analysing, integrating, processing and reporting data on drugs performance and interactions in relation to related data of the patient which can be able to predict trends in side effects, drug interactions, and potential issues with respect to various drugs before their release to the patients.

The use of the invention will have the advantage of providing an effective alert for physicians or pharmacists on problematic drugs, limiting the number of people affected by such drugs, also enabling pharmaceutical companies to begin improving drugs sooner, and substantially lowering health-related costs.

Hence, an improved and more efficient method for collecting, integrating and analysing and processing data from a variety of sources so as to generate drugs interaction analyses and reports would be advantageous.

### OBJECT OF THE INVENTION

It is an object of the invention to provide a system and method for collecting, integrating and analysing and processing data from a variety of sources so as to generate drugs interaction analyses and reports information to provide doctors, physicians, pharmacists, general practitioners and patients with information on drug interaction and drug interaction effects.

It is a further object of the present invention to provide a more convenient, simple and effective alternative to the prior art.

In particular, it may be seen as an object of the present invention to provide a more reliable method for collecting, integrating and analysing and processing data from a variety of sources so as to generate drugs interaction analyses and reports that solves the above mentioned problems of the prior art.

### SUMMARY OF THE INVENTION

The database has been developed in order to inform doctors and pharmacists of potential drug interactions with patients who have been prescribed one or more different drugs. The database would enable physicians and pharmacists get efficient and brief information on the possible interactions.

The invention is particularly, but not exclusively, advantageous for providing doctors, physicians, pharmacists, general practitioners with a tool assisting their decisions on: whether to continue treatment with a potentially harmful combination; whether to replace the relevant drug with some other with no interactions; whether to check upon the patient during the treatment; whether to change dosages; whether to inform the patient on the interactions.

Moreover the invention is particularly but not exclusively, advantageous for providing a more reliable and significant tool in the evaluation of drug-drug interaction (DDI). For the purpose of the invention drug-drug interactions are also referred as drugs interactions within the text.

Thus, the above described object and several other objects are intended to be obtained in a first aspect of the invention by providing a method for collecting, integrating, analysing and processing medical information, the method comprising: applying inclusion and exclusion criteria in the collection of information regarding drugs interactions; analysing drug interactions based on the collected information; reporting the drugs interaction in an easily readable and searchable format.

In some embodiments the application of criteria of criteria comprise: dividing all drugs interactions into a least two groups and assigning different criteria for said at least two groups.

All drug interactions may be divided into at least two groups, e.g. pharmacodynamic interactions and pharmacokinetic interactions.

Pharmacodynamic interactions may be further divided into at least two subgroups, e.g. predictable and unpredictable. Predictable interactions are drugs interaction based on pharmacodynamic properties of drugs with interactions, e.g. additive interactions, potentiating of effects or antagonism. Unpredictable interactions are interactions which are not linked to the known pharmacodynamic properties of drugs with interactions.

Pharmacokinetic interactions may be for example:
1) absorption, chelation and other complex-forming mechanisms;
2) changes in the motility of the gastrointestinal tract;
3) induction or inhibition of the drug's transport proteins;
4) drug-induced malabsoption;
5) interactions due to coupling with proteins;
6) metabolization of the drug, such as
   i. fist-pass metabolism
   ii. induction of enzymes
   iii. inhibition of enzymes
   iv. CYP enzymes and predictable drug interactions;
7) interactions due to drug secretion.

In some embodiments the information are collected by searching in at least one database.

In collecting information regarding drugs interactions a priori selection is made based on the database used for search of information regarding drugs interactions. For example drugs interactions information may be collected from public databases, such as FDA databases, British National Formulary. Drugs interactions information may be also collected from publications regarding interactions retrieved from sources of biomedical bibliographic information such as Medline.

The collected publications may be divided in several different collections, e.g. clinical studies, case report, Metanalysis articles, cohort study articles and reviews.

Collected information regarding drug interaction can be based on interaction between pharmaceutical compositions, between active ingredients of drugs or between active ingredient of one drug and other non-active ingredients of another drug.

All collected information are considered substantial if they fulfil inclusion criteria which depend on the types of drug interactions.

Assigning inclusion and exclusion criteria on the collection of information regarding drugs interaction has the advantage of selecting significant scientific information regarding the evaluation of drug interactions "a priori", i.e. before the risk assessment is performed. In this way the assessment and evaluation of controversial information is facilitated as only the significant information are contained in the collection. For example if one single report on drug interaction is retrieved, its significance may be so that the information can be excluded from the collection of the information. Accordingly the gravity of the symptoms due to drugs interactions is taken into consideration. For a single case of drug interaction reporting headaches has a different significance and therefore importance in the collection of information than a single case of death alleged to be due to drugs interaction.

Assigning different criteria for the different groups has the advantage of producing a more correct and reliable collection of information as for different type of drug interaction different type of inclusion and exclusion criteria maybe more relevant. The method has as advantage the introduction of a step of selection of the data available so that the most relevant and significant data are chosen leading to a correct assessment of DDIs and a high reliability of results reported.

When the collected information relates to a pharmacodynamic drug interaction inclusion criteria maybe:
1) Theoretically predictable synergistic interactions
   i. Anticholinergic activity
   ii. Antihypertensive activity
   iii. Hypokalemia
   iv. Psychomotoric disorders, sedation
   v. Drugs extending the QT-interval (increased torsades de pointes risk)
   vi. Bone marrow megaloblastosis
   vii. Increased nephrotoxicity
   viii. Increased neuromuscular blockade
   ix. Hyperkalemia
   x. Increased negative isotropic activity
2) Theoretically predictable antagonistic interactions
   i. Interactions between anticoagulants and vitamin K
   ii. Antidiabetic drugs and glycocorticoids
   iii. Antitumor agents and megestrol
   iv. Levodopa and antipsychotics
   v. Levodopa and tacrine
3) information of two or more interaction cases retrieved from sources of biomedical bibliographic information, e.g. from Medline, together information collected from other sources, e.g. metanalysis, reviews, reference books, public databases of drug interactions
4) one or more clinical researches with or without case reports retrieved from sources of biomedical bibliographic information, e.g. from Medline.

When the collected information relates to a pharmacodynamic drug interaction exclusion criteria maybe:
1) if less than two cases are reported,
2) if interactions appears within drugs of the same group, e.g. two anticholinergic agents, two phenothiazine derivatives, two benzodiazepines,
3) if interaction cases are due to drug poisoning, e.g. overdose,
4) if interactions theoretically predictable results in negative data from more than one study.

When the collected information relates to a pharmacokinetic drug interaction inclusion criteria maybe:
1) Theoretically predictable interactions confirmed by data regarding two or more cases
2) Theoretically predictable interactions confirmed by at least one clinical study
3) Interactions referred to as "clinically important" in the IDE database
4) Clinically recorded interactions in clinical studies and/or one or more reported case reports.

When the collected information relates to a pharmacokinetic drug interaction exclusion criteria maybe:
1) if less than two case are reported,
2) if interactions appears within drugs of the same group,
3) if interaction cases are due to drug poisoning, e.g. overdose,
4) if theoretical considerations are not supported by clinical studies and there are no records of cases reported in sources of biomedical bibliographic information such as Medline.

In some embodiments the analysis of drugs interactions comprises assessing the risk of drugs interactions.

The collected information is analyzed and interpreted taking in consideration: dosage used, term of treatment, age, sex and associated illnesses related to the patients, frequency of interactions.

In some other embodiments the assessment of the risk of drugs interactions is carried out by a scientist.

In some embodiments the analysis comprises the evaluation of controversial information.

The presence of controversial information is evaluated taking in consideration a number of positive signals, a number of negative signals, the used dosages, manufacturers considerations, metanalysis data, information from reference books, EDI monographs (Evaluations of Drug Interactions), Stockley's Drug Interactions monographs.

One of the advantages of valuating controversial information provides more reliable reports on drugs interaction

In some embodiments the reporting comprises a reference to Drug ATC code.

In some other embodiments the reporting comprises a reference to active ingredient, e.g. in Latin language.

In some embodiments the reporting comprises a description of the drugs interactions.

In some other embodiments the reporting comprises a factor of importance, such as a coloured degree of importance.

Reporting of drugs interaction is in the form of monograph for the different drugs and/or active principle. The monographs are edited by scientists who generate conclusions based on the large amount of scientific data collected by applying the criteria previously mentioned. This has the advantage of providing a higher quality and reliability of the information regarding the information regarding drugs interactions provided though the database. In this way a large amount of data are used to generate a simple report on drugs interaction.

The monographs are available in a user friendly interface so that information about drugs interaction can be easily search, accessed and understood.

For example the output of the monographs through the user friendly interface may comprise references to the drug ATC code, so as to easily identify the drug even in medicine with different commercial names. In some other embodiments the output of the monographs through the user friendly interface may also comprise references to active ingredient, in Latin, which may also have the advantage of avoiding potential mistakes in the analysis due to the different languages of the information collected. In some other embodiments the output of the monographs through the user friendly interface may comprise a description of drugs interactions, e.g. the physiological effect of the drug.

In some other embodiments the output of the monographs through the user friendly interface may comprise suggestions on how to avoid drugs interaction, e.g. by suggestion a substitute to the drug which may produce undesired interactions or by suggesting specific dosage which may provide compatibility or even suggesting a possible additional product that will balance and/or eliminate the incompatibility between the two mentioned two drugs.

In some other embodiments the output of the monographs through the user friendly interface may optionally comprise a factor of importance or significance of the drugs interaction.

For example in some embodiments of the invention a colour code is used to identify the degree of incompatibility in a use of a specific combination of drugs.

For example a pharmacist collecting drugs to be delivered to a patient may access the database with an interface showing a colour code. For instance while retrieving drug A, a red flag with drugs B and C may appear, while a yellow flag with drug D may appear. This will make the pharmacist aware that providing the patient with a combination of drugs A, B or C may have high risk of drugs interaction, while providing the patient with drugs A and D may have less risk of interactions.

The previously described object and several other objects are intended to be obtained in a second aspect of the invention by a computer based system for collecting, integrating, analysing and processing medical information the system comprising: at least one electronic data processor; a data communication interface connected with said processor for accessing a plurality of databases containing drugs interaction information; a situational modeller configured to execute said electronic processor, in which said situational modeller is configured to: apply inclusion and exclusion criteria in the collection of information regarding drugs interactions; analyse drugs interactions based on the collected information; report the drugs interaction in an easily readable and searchable format.

The previously described object and several other objects are intended to be obtained in another aspect of the invention by a computer-readable storage medium having stored therein computer readable instructions, which, when loaded in and executed by a computer causes the computer to perform the steps of: applying inclusion and exclusion criteria in collection of information regarding drugs interactions from a plurality of database containing drugs interaction information; analysing drug interactions based on the collected information; reporting said drugs interactions in an easily readable and searchable format.

The first, second and other aspect of the present invention may each be combined with any of the other aspects. These and other aspects of the invention will be apparent from and elucidated with reference to the embodiments described hereinafter.

### BRIEF DESCRIPTION OF THE FIGURES

The method according to the invention will now be described in more detail with regard to the accompanying figures. The figures show one way of implementing the present invention and is not to be construed as being limiting to other possible embodiments falling within the scope of the attached claim set.
Figure 1 is a flow-chart of the method according to the invention.
Figure 2 is an example of the user interface containing information from the monographs reporting drugs interactions.

### DETAILED DESCRIPTION OF AN EMBODIMENT

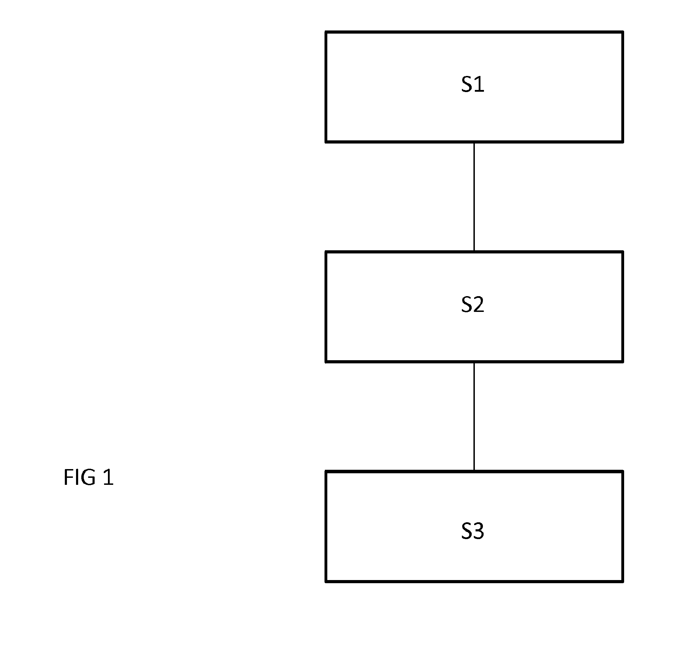

Figure 1 is a flow-chart of the method according to the invention.

The flowchart shows the steps of applying inclusion and exclusion criteria in the collection of information regarding drugs interactions (S1), analysing drug interactions based on the collected information (S2) and (S3) reporting the drugs interaction in an easily readable and searchable format.

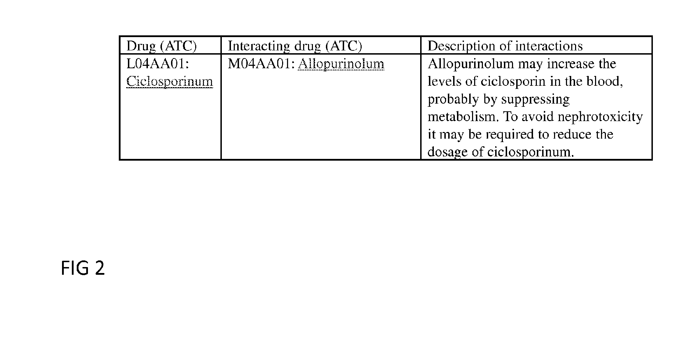

Figure 2 is an example of the user interface containing information from the monographs reporting drugs interactions.

The invention may be realized in hardware, software, or a combination of hardware and software. The invention may be realized in a centralized manner in one computer system, or in a distributed manner where different elements are spread across several interconnected computer systems. Any type of computer system or other apparatus adapted for carrying out the methods described herein is suitable. A typical combination of hardware and software can be a general purpose computer system with a computer program that, when being loaded and executed, controls the computer system such that it carries out the methods described herein.

The invention may be embedded in a computer program product, such as magnetic tape, an optically readable disk, or other computer-readable medium for storing electronic data. The computer program product may comprise computer-readable code, defining a computer program, which when loaded in a computer or computer system causes the computer or computer system to carry out the different methods described herein. Computer program in the present context means any expression, in any language, code or notation, of a set of instructions intended to cause a system having an information processing capability to perform a particular function either directly or after either or both of the following:
a) conversion to another language, code or notation; b) reproduction in a different material form.

The preceding description of preferred embodiments of the invention has been presented for the purposes of illustration. The description provided is not intended to limit the invention to the particular forms disclosed or described. Modifications and variations will be readily apparent from the preceding description. As a result, it is intended that the scope of the invention not be limited by the detailed description provided herein.

Although the present invention has been described in connection with the specified embodiments, it should not be construed as being in any way limited to the presented examples. The scope of the present invention is set out by the accompanying claim set. In the context of the claims, the terms "comprising" or "comprises" do not exclude other possible elements or steps. Also, the mentioning of references such as "a" or "an" etc. should not be construed as excluding a plurality. The use of reference signs in the claims with respect to elements indicated in the figures shall also not be construed as limiting the scope of the invention. Furthermore, individual features mentioned in different claims, may possibly be advantageously combined, and the mentioning of these features in different claims does not exclude that a combination of features is not possible and advantageous.

## Claims

1. A method for collecting, integrating, analysing and processing medical information, the method comprising,
- applying inclusion and exclusion criteria in the collection of information regarding drugs interactions
- analysing drug interactions based on the collected information
- reporting the drugs interaction in an easily readable and searchable format.

2. A method according to claim 1, wherein said information are collected by searching in at least one database.

3. A method according to claims 1-2, wherein the application of criteria of criteria comprise:
- Dividing all drugs interactions into a least two groups
- Assigning different criteria for said at least two groups.

4. A method according to claim 3, wherein said at least two groups comprise pharmacodynamic interactions and/or pharmacokinetic interactions.

5. A method according to claim 3-4, wherein the inclusion criteria for a group comprise including information if one or more clinical researches with or without case reports are retrieved from sources of biomedical bibliographic information.

6. A method according to claim 3-5, wherein the inclusion criteria for another group comprise including information if clinically recorded interactions in clinical studies and/or one or more reported case reports.

7. A method according to claim 3-6, wherein the exclusion criteria for a group comprise excluding information if less than two cases are reported.

8. A method according to claim 3-7, wherein the exclusion criteria for another group comprise excluding information if theoretical considerations are not supported by clinical studies.

9. A method according to any of the preceding claims wherein the analysis comprise assessing the risk of drugs interactions.

10. A method according to any of the preceding claims wherein the analysis comprises the assessment the risk of drugs interactions is carried out by a scientist.

11. A method according to any of the preceding claims wherein the analysis comprises the evaluation of controversial information.

12. A method according to any of the preceding claims wherein said reporting comprises a reference to active ingredient.

13. A method according to any of the preceding claims wherein said reporting comprises a description of the drugs interactions.

14. A computer based system for collecting, integrating, analysing and processing medical information the system comprising:
- at least one electronic data processor
- a data communication interface connected with said processor for accessing a plurality of databases containing drugs interaction information;
- a situational modeller configured to execute said electronic processor, in which said situational modeller is configured to:
• apply inclusion and exclusion criteria in the collection of information regarding drugs interactions
• analyse drugs interactions based on the collected information
• report the drugs interaction in an easily readable and searchable format.

15. A computer-readable storage medium having stored therein computer readable instructions, which, when loaded in and executed by a computer causes the computer to perform the steps of:
• applying inclusion and exclusion criteria in collection of information regarding drugs interactions from a plurality of database containing drugs interaction information
• analysing drug interactions based on the collected information
• reporting said drugs interactions in an easily readable and searchable format.
